(19) Europäisches Patentamt
European Patent Office
Office européen des brevets

(11) Veröffentlichungsnummer: **0 325 708 B1**

# (12) EUROPÄISCHE PATENTSCHRIFT

(45) Veröffentlichungstag der Patentschrift: **18.03.92**

(51) Int. Cl.[5]: **C07D 209/08**, C07D 209/10, C07D 209/12, C07D 209/24, C07D 209/22

(21) Anmeldenummer: **88118359.4**

(22) Anmeldetag: **04.11.88**

## (54) Bis-indolyl-ethylen-Derivate.

(30) Priorität: **11.11.87 DE 3738237**

(43) Veröffentlichungstag der Anmeldung:
**02.08.89 Patentblatt 89/31**

(45) Bekanntmachung des Hinweises auf die Patenterteilung:
**18.03.92 Patentblatt 92/12**

(84) Benannte Vertragsstaaten:
**CH DE FR GB LI**

(56) Entgegenhaltungen:
**FR-A- 2 259 883**

**JOURNAL OF HETEROCYCLIC CHEMISTRY, Band 22, Nr. 2, März-April 1985, Seiten 341-343; J. BERGMAN: "Reactions of indoles with ortho esters,N,N-dimethylformamide and N,N-dimenthylacetamide dialkyl acetals"**

(73) Patentinhaber: **BAYER AG**

**W-5090 Leverkusen 1 Bayerwerk(DE)**

(72) Erfinder: **Psaar, Hubertus, Dr.**
**Paul-Klee-Strasse 21**
**W-5090 Leverkusen 1(DE)**
Erfinder: **Berneth, Horst, Dr.**
**Erfurter Strasse 1**
**W-5090 Leverkusen 1(DE)**

**EP 0 325 708 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Bis-indolyl-ethylenen der Formel

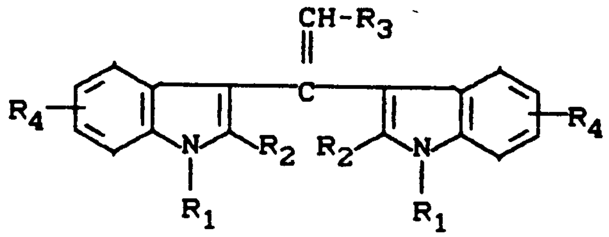

(I)

worin

$R_1$ Wasserstoff, Alkyl, Aralkyl oder Aryl,
$R_2$ Wasserstoff, Alkyl oder Aryl,
$R_3$ Wasserstoff, Alkyl, Alkenyl, COOH oder Aryl und
$R_4$ Wasserstoff, Alkyl, Alkoxy, Cycloalkoxy oder Halogen bedeuten, und

diese Substituenten durch Halogen, $C_1$- bis $C_4$-Alkoxy, Cyan oder eine Carboxylgruppe und cyclische Substituenten zusätzlich durch $C_1$- bis $C_4$-Alkyl substituiert sein können,
durch Umsetzung von Indolen der Formel

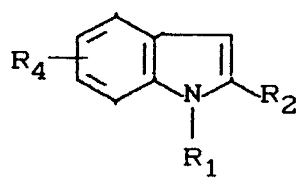

(II)

mit Carbonsäuren der Formel

$R_3$-$CH_2$-COOH (III)

ihren Chloriden, Estern, beispielsweise Alkylestern oder Anhydriden in Anwesenheit von Phosphoroxychlorid.

Bevorzugt stehen Alkoxy für $C_1$-$C_{12}$-Alkoxy, Alkyl für $C_1$-$C_{12}$-Alkyl, Alkenyl für $C_2$-$C_{12}$-Alkenyl, Aryl für Phenyl, Aralkyl für Benzyl oder Phenylethyl, Cycloalkoxy für Cyclohexoxy oder Cyclopentoxy und Halogen für Fluor, Chlor oder Brom, insbesondere Chlor.

Die bevorzugte Reaktionstemperatur liegt bei 50 °C bis 105 °C, und die bevorzugte Reaktionszeit liegt zwischen 20 Minuten und 3 Stunden.
Das Molverhältnis von (II) : (III) soll vorzugsweise höchstens 2:1 betragen.

Phosphoroxychlorid wird vorteilhafterweise im Molverhältnis von 1,5 bis 2:1 bezogen auf (III) oder deren Derivate eingesetzt.

Die Umsetzung kann mit oder ohne Lösungsmittel durchgeführt werden, wobei im letzteren Fall die Menge an Phosphoroxychlorid so bemessen sein soll, daß dieses gleichzeitig als Lösungsmittel wirkt. Als Lösungsmittel können Aromaten, alkylierte Aromaten oder chlorierte Aromaten eingesetzt werden. Geeignete Lösungsmittel sind z.B. Toluol, Xylol, Chlorbenzol oder Dichlorbenzol.

Das Verfahren ermöglicht die Herstellung der Verbindungen (I) in einfacher Weise mit hoher Ausbeute.

Ein weiterer Gegenstand der Erfindung sind Bis-indolyl-ethylene der Formel

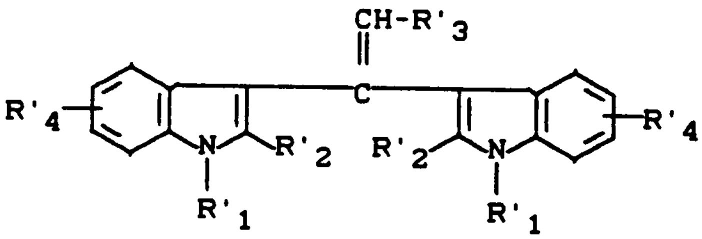

(IV)

worin
entweder

a)

| | |
|---|---|
| $R'_1$ | $C_2$-$C_{12}$-Alkyl oder Benzyl, |
| $R'_2$ und $R'_3$ | Wasserstoff, Alkyl oder Benzyl, |
| $R'_4$ | Wasserstoff, Alkyl, Alkoxy oder Halogen bedeuten, und Phenyl und Benzyl, die durch Alkyl, Alkoxy oder Halogen substituiert sein können, |

oder

b)

| | |
|---|---|
| $R'_1$ | Wasserstoff, Alkyl oder Benzyl, das durch Alkyl, Alkoxy oder Halogen substituiert sein kann, und |
| $R'_4$ | Alkyl, Alkoxy oder Halogen bedeuten, und |
| $R'_2$ und $R'_3$ | die obengenannte Bedeutung besitzen, |

oder

c)

| | |
|---|---|
| $R'_1$ | Wasserstoff, Alkyl oder Benzyl, das durch Alkyl, Alkoxy oder Halogen substituiert sein kann, |
| $R'_2$ | durch Alkyl, Alkoxy oder Halogen substituiertes Phenyl, |
| $R'_3$ | Wasserstoff, Alkyl oder Phenyl, das durch Alkyl, Alkoxy oder Halogen substituiert sein kann, und |
| $R'_4$ | Wasserstoff, Alkyl, Alkoxy oder Halogen bedeuten, |

oder

d)

| | |
|---|---|
| $R'_1$ | Wasserstoff, Alkyl oder Benzyl, das durch Alkyl, Alkoxy oder Halogen substituiert sein kann, |
| $R'_2$ | Phenyl, |
| $R'_3$ | Alkyl oder Phenyl und |
| $R'_4$ | Wasserstoff, Alkyl, Alkoxy oder Halogen bedeuten, |

und worin Alkyl - wenn nicht besonders benannt -für $C_1$-$C_{12}$-Alkyl und Alkoxy für $C_1$-$C_{12}$-Alkoxy stehen und die Alkylreste durch Chlor, Cyan oder Carboxy substituiert sein können.

Besonders bevorzugt sind die Verbindungen c) und d).

Die Verbindungen der Formel (I) sind wertvolle Zwischenprodukte für Farbbildner, die z. B. in DE-A-37 38 240 beschrieben werden.

Beispiel 1

20, 7 Gew.-Teile 1-Methyl-2-phenyl-indol werden in 30 ml Phosphoroxychlorid auf 80 bis 90° C unter Rühren erhitzt. Bei dieser Temperatur werden 5, 3 Gew.-Teile Essigsäureanhydrid zugetropft und der Ansatz 30 Minuten bei 100° C gerührt. Die Mischung wird dann auf 200 Gew.-Teile Eiswasser ausgetragen, mit 10 %iger Natronlauge alkalisch gestellt, 15 Stunden bei Raumtemperatur gerührt, abgesaugt und aus Dimethylformamid umkristallisiert.

Ausbeute: 20,3 Gew.-Teile; F: 192-194° C.

Die Verbindung besitzt die Formel

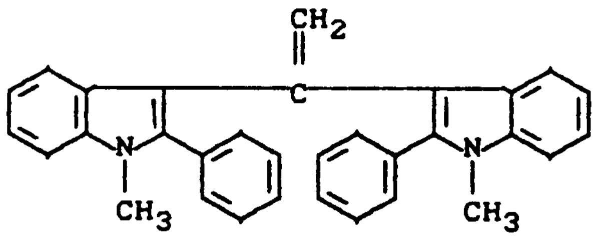

Beispiel 2

20,7 Gew.-Teile 1-Methyl-2-phenyl-indol werden in 50 ml Phosphoroxychlorid auf 90° C erhitzt und bei dieser Temperatur 4,9 Gew.-Teile Propionsäure zugetropft. Nach 30 Minuten wird auf Raumtemperatur abgekühlt und 100 ml Methanol langsam zugegeben. Anschließend wird der Ansatz in eine Mischung von 500 ml konz. Ammoniak und 500 ml Wasser eingerührt. Das Produkt wird abgesaugt, mit Methanol verrührt und bei 50° C im Vakuum getrocknet.
Ausbeute: 22,1 Gew.-Teile
F.: 184-186° C
Die Verbindung hat die Formel

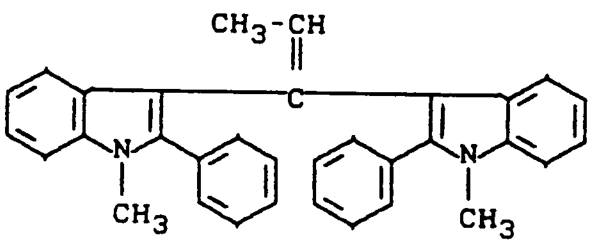

Beispiel 3

Tropft man anstelle von Propionsäure gemäß Beispiel 2 10 Gew.-Teile Butyrolacton zum Ansatz, so erhält man das Bis-indolyl-ethylen der Formel

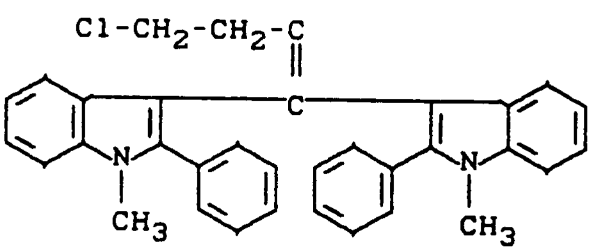

Ausbeute: 24,5 Gew.-Teile
F.: 101-103° C
Analog Beispiel 1 wurden die Verbindungen der Formel

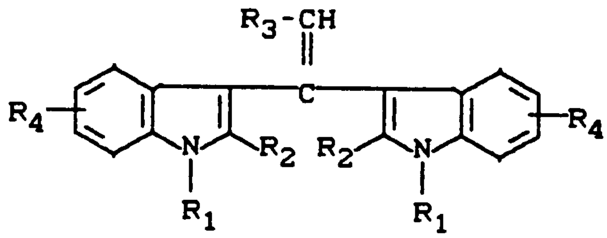

hergestellt.

| Beispiel | $R_1$ | $R_2$ | $R_3$ | $R_4$ | F. |
|---|---|---|---|---|---|
| 4 | $CH_3$ | $CH_3$ | H | H | 173 °C |
| 5 | $CH_3$ | H | H | H | 142-143 °C |
| 6 | $CH_3$ | $CH_3$ | $CH_3$ | H | 101-103 °C |
| 7 | $C_4H_9$ | $CH_3$ | H | H | |
| 8 | $CH_3$ | $CH_3$ | $C_{10}H_{21}$ | H | 97-99 °C |
| 9 | $CH_3$ | $C_6H_5$ | $CH_2$-COOH | H | 108-112 °C |
| 10 | $CH_3$ | $C_6H_5$ | $CH=CH_2$ | H | 165-167 °C |
| 11 | $C_2H_4CN$ | $C_6H_5$ | H | H | 210 °C |
| 12 | $C_2H_4COOH$ | $C_6H_5$ | H | H | 220 °C |
| 13 | $C_4H_9$ | $C_6H_5$ | H | H | 89-91 °C |
| 14 | $C_6H_5CH_2$ | $C_6H_5$ | H | H | 238-240 °C |
| 15 | $C_6H_5CH_2$ | $CH_3$ | H | H | 148-149 °C |
| 16 | $CH_3$ | $C_6H_4$, 4-$OCH_3$ | H | H | 172-174 °C |
| 17 | $CH_3$ | $C_6H_4$, 4-Cl | H | H | 210 °C |
| 18 | $CH_3$ | $C_6H_5$ | H | 6-Cl | 214-217 °C |
| 19 | $CH_3$ | $C_6H_5$ | H | 5-$CH_3$ | 225-227 °C |
| 20 | $C_2H_5$ | $C_6H_5$ | H | H | 160 °C |
| 21 | $CH_3$ | $C_6H_5$ | Cl | H | 182,5 °C |
| 22 | H | $C_6H_5$ | H | H | 244-246 °C |
| 23 | H | $C_6H_5$ | $CH_3$ | H | 169 °C |
| 24 | $C_6H_5CH_2$ | $C_6H_5$ | $CH_3$ | H | 80 °C |

## Patentansprüche

**1.** Verfahren zur Herstellung von Bis-indolyl-ethylenen der Formel

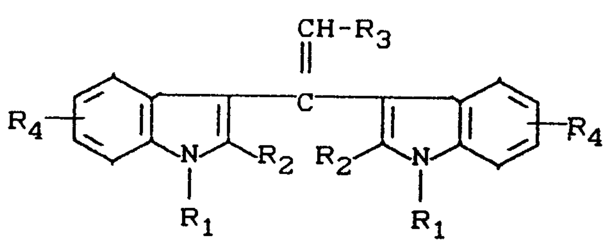

worin

$R_1$ Wasserstoff, Alkyl, Aralkyl oder Aryl,
$R_2$ Wasserstoff, Alkyl oder Aryl,
$R_3$ Wasserstoff, Alkyl, Alkenyl, COOH oder Aryl und
$R_4$ Wasserstoff, Alkyl, Alkoxy, Cycloalkoxy oder Halogen bedeuten, und

diese Substituenten ihrerseits durch Halogen, $C_1$-bis $C_4$-Alkoxy, Cyan oder eine Carboxylgruppe und cyclische Substituenten zusätzliche durch $C_1$- bis $C_4$-Alkyl substituiert sein können,

durch Umsetzung von Indolen der Formel

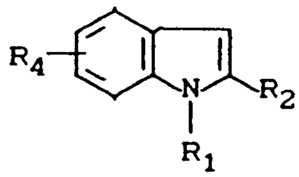

mit Carbonsäuren der Formel

$R_3$-$CH_2$-COOH

ihren Chloriden, Estern, beispielsweise Alkylestern oder Anhydriden in Anwesenheit von Phosphoroxychlorid.

**2.** Verfahren nach Anspruch 1, dadurch gekennzeichnet, daß man die Umsetzung bei 50 °C bis 105 °C während 20 Minuten bis 3 Stunden durchführt.

**3.** Verfahren nach Anspruch 1, dadurch gekennzeichnet, daß man die Umsetzung mit einem Molverhältnis von Phosphoroxychlorid zu Carbonsäure oder ihren Derivaten von 1,5 bis 2:1 durchführt.

**4.** Bis-indolyl-ethylene der Formel

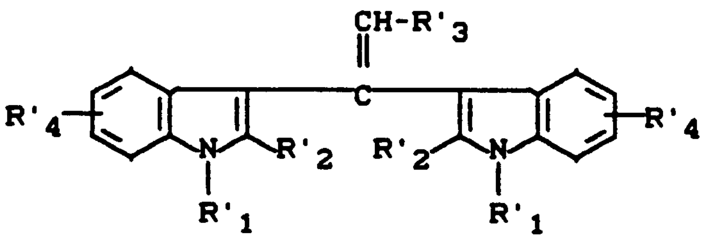

worin
entweder
a)
$R'_1$ $C_2$-$C_{12}$-Alkyl oder Benzyl,
$R'_2$ und $R'_3$ Wasserstoff, Alkyl oder Benzyl,
$R'_4$ Wasserstoff, Alkyl, Alkoxy oder Halogen bedeuten, und Phenyl und Benzyl, die durch Alkyl, Alkoxy oder Halogen substituiert sein können,
oder
b)
$R'_1$ Wasserstoff, Alkyl oder Benzyl, das durch Alkyl, Alkoxy oder Halogen substituiert sein kann, und
$R'_4$ Alkyl, Alkoxy oder Halogen bedeuten, und
$R'_2$ und $R'_3$ die obengenannte Bedeutung besitzen,
oder
c)
$R'_1$ Wasserstoff, Alkyl oder Benzyl, das durch Alkyl, Alkoxy oder Halogen substituiert sein kann,
$R'_2$ durch Alkyl, Alkoxy oder Halogen substituiertes Phenyl,
$R'_3$ Wasserstoff, Alykl oder Phenyl, das durch Alkyl, Alkoxy oder Halogen substituiert sein kann, und
$R'_4$ Wasserstoff, Alkyl, Alkoxy oder Halogen bedeuten,
oder
d)

$R'_1$ Wasserstoff, Alkyl oder Benzyl, das durch Alkyl, Alkoxy oder Halogen substituiert sein kann,
$R'_2$ Phenyl,
$R'_3$ Alkyl oder Phenyl und
$R'_4$ Wasserstoff, Alkyl, Alkoxy oder Halogen bedeuten,
und worin Alkyl - wenn nicht besonders benannt - für $C_1$-$C_{12}$-Alkyl und Alkoxy für $C_1$-$C_{12}$-Alkoxy stehen und die Alkylreste durch Chlor, Cyan oder Carboxy substituiert sein können.

**5.** Bis-indolyl-ethylene nach Anspruch 4, dadurch gekennzeichnet, daß
$R'_1$ Wasserstoff, Alkyl oder Benzyl,
$R'_2$ durch Alkyl, Alkoxy oder Halogen substituiertes Phenyl,
$R'_3$ Wasserstoff, Alkyl oder Phenyl, und
$R'_4$ Wasserstoff, Alkyl, Alkoxy oder Halogen bedeuten, und die Alkyl - und Alkoxy-Reste 1 bis 12 C-Atome haben.

**6.** Bis-indolyl-ethylene nach Anspruch 4, dadurch gekennzeichnet, daß
$R'_1$ Wasserstoff, Alkyl oder Benzyl,
$R'_2$ Phenyl,
$R'_3$ Alkyl oder Phenyl und
$R'_4$ Wasserstoff, Alkyl, Alkoxy oder Halogen bedeuten, und die Alkyl- und Alkoxy-Reste 1-12 C-Atome haben.

## Claims

**1.** Process for the preparation of bis(indolyl)ethylenes of the formula

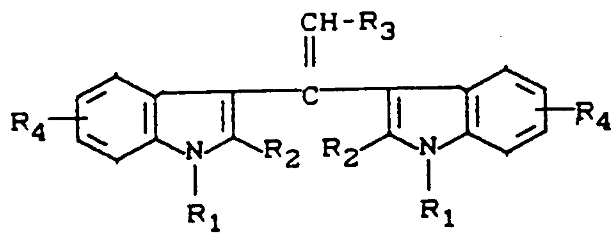

wherein
$R_1$ denotes hydrogen, alkyl, aralkyl or aryl,
$R_2$ denotes hydrogen, alkyl or aryl,
$R_3$ denotes hydrogen, alkyl, alkenyl, COOH or aryl and
$R_4$ denotes hydrogen, alkyl, alkoxy, cycloalkoxy or halogen, and
these substituents, in turn, can be substituted by halogen, $C_1$- to $C_4$-alkoxy, cyano or a carboxyl group and cyclic substituents can additionally be substituted by $C_1$-to $C_4$-alkyl,
by reaction of indoles of the formula

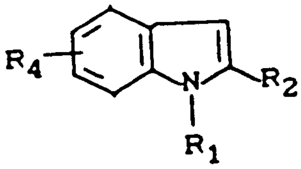

with carboxylic acids of the formula

$R_3$-$CH_2$-COOH

their chlorides, esters, for example alkyl esters, or anhydrides in the presence of phosphorus oxychloride.

2. Process according to Claim 1, characterised in that the reaction is carried out at 50 ° C to 105 ° C during the course of 20 minutes to 3 hours.

3. Process according to Claim 1, characterised in that the reaction is carried out using a molar ratio of phosphorus oxychloride to carboxylic acid or its derivatives of 1.5 to 2:1.

4. Bis(indolyl)ethylenes of the formula

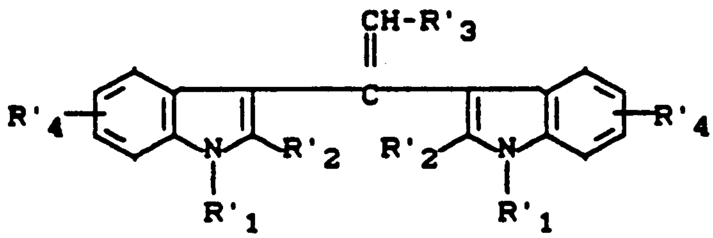

wherein
either
a) $R'_1$ denotes $C_2$-$C_{12}$-alkyl or benzyl,
$R'_2$ and $R'_3$ denote hydrogen, alkyl or benzyl,
$R'_4$ denotes hydrogen, alkyl, alkoxy or halogen, and phenyl and benzyl, each of which can be substituted by alkyl, alkoxy or halogen,
or,
b) $R'_1$ denotes hydrogen, alkyl, or benzyl which can be substituted by alkyl, alkoxy or halogen, and
$R'_4$ denotes alkyl, alkoxy or halogen, and
$R'_2$ and $R'_3$ have the abovementioned meaning,
or
c) $R'_1$ denotes hydrogen, alkyl, or benzyl which can be substituted by alkyl, alkoxy or halogen,
$R'_2$ denotes phenyl which can be substituted by alkyl, alkoxy or halogen,
$R'_3$ denotes hydrogen, allyl, or phenyl which can be substituted by alkyl, alkoxy or halogen, and
$R'_4$ denotes hydrogen, alkyl, alkoxy or halogen,
or
d) $R'_1$ denotes hydrogen, alkyl, or benzyl which can be substituted by alkyl, alkoxy or halogen,
$R'_2$ denotes phenyl,
$R'_3$ denotes alkyl or phenyl and
$R'_4$ denotes hydrogen, alkyl, alkoxy or halogen, and wherein alkyl - when not named separately - represents $C_1$-$C_{12}$-alkyl and alkoxy represents $C_1$-$C_{12}$-alkoxy and the alkyl radicals can be substituted by chlorine, cyano or carboxyl.

5. Bis(indolyl)ethylenes according to Claim 4, characterised in that
$R'_1$ denotes hydrogen, alkyl or benzyl,
$R'_2$ denotes phenyl which is substituted by alkyl, alkoxy or halogen,
$R'_3$ denotes hydrogen, alkyl or phenyl, and
$R'_4$ denotes hydrogen, alkyl, alkoxy or halogen, and the alkyl and alkoxy radicals have 1 to 12 C atoms.

6. Bis(indolyl)ethylenes according to Claim 4, characterised in that
$R'_1$ denotes hydrogen, alkyl or benzyl,
$R'_2$ denotes phenyl,
$R'_3$ denotes alkyl or phenyl and
$R'_4$ denotes hydrogen, alkyl, alkoxy or halogen, and the alkyl and alkoxy radicals have 1 to 12 C atoms.

**Revendications**

1. Procédé pour préparer des bis-indolyl-éthylènes de formule :

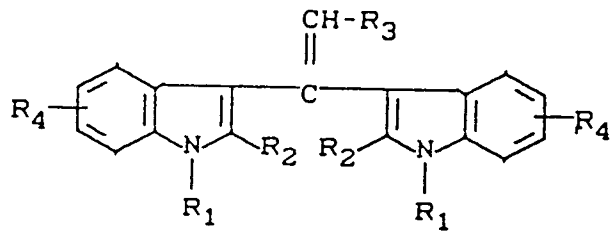

dans laquelle

$R_1$ représente un atome d'hydrogène, un groupe alkyle, aralkyle ou aryle,

$R_2$ représente un atome d'hydrogène, un groupe alkyle ou aryle,

$R_3$ représente un atome d'hydrogène, un groupe alkyle, alcényle, COOH ou aryle, et

$R_4$ représente un atome d'hydrogène un groupe alkyle, alcoxy, cycloalcoxy ou halogéno, et

ces substituants peuvent être pour leur part substitués par de l'halogène, par un reste alcoxy en $C_1$ à $C_4$ cyano, ou bien un groupe carboxyle et des substituants cycliques peuvent être en outre être substitués par des restes alkyles en $C_1$ à $C_4$, par réaction d'indoles de formule :

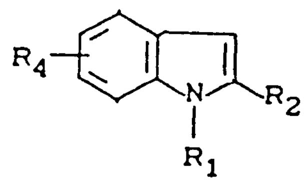

avec des acides carboxyliques de formule :

$R_3$-$CH_2$-COOH

leurs chlorures, esters, par exemple leurs esters alkyliques ou leurs anhydrides, en présence de l'oxychlorure de phosphore.

**2.** Procédé selon la revendication 1, caractérisé en ce qu'on conduit la réaction à une température de 50 ° C à 105 ° C pendant 20 min à 3 h.

**3.** Procédé selon la revendication 1, caractérisé en ce qu'on conduit la réaction en utilisant un rapport molaire entre l'oxychlorure de phosphore et l'acide carboxylique ou ses dérivés qui va de 1,5 à 2:1.

**4.** Bis-indolyl-éthylènes de formule :

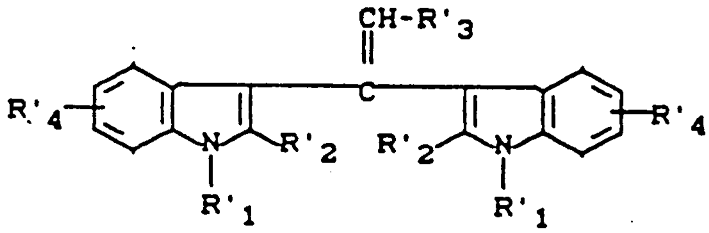

dans laquelle

a)

$R'_1$ représente un groupe alkyle en $C_2$ à $C_{12}$ ou un groupe benzyle,

$R'_2$ et $R'_3$ représentent chacun un atome d'hydrogène ou un groupe alkyle ou benzyle,

$R'_4$ représente un atome d'hydrogène, un groupe alkyle, alcoxy ou halogéno, et phényle et benzyle, qui peuvent être substitués par un reste alkyle, alcoxy ou

halogéno,

ou bien

b)

R'$_1$ représente un atome d'hydrogène, un groupe alkyle ou benzyle, qui peuvent être substitué par un reste alkyle, alcoxy ou halogéno, et

R'$_4$ représente un groupe alkyle, alcoxy ou halogéno, et

R'$_2$ et R'$_3$ ont le sens indiqué ci-dessus,

ou bien

c)

R'$_1$ représente un atome d'hydrogène, un groupe alkyle ou benzyle, qui peuvent être substitué par un reste alkyle, alcoxy ou halogéno,

R'$_2$ représente un groupe phényle substitué par un reste alkyle, alcoxy ou halogéno,

R'$_3$ représente un atome d'hydrogène, un groupe alkyle ou phényle, qui peut être substitué par un reste alkyle, alcoxy ou halogéno, et

R'$_4$ représente un atome d'hydrogène, un groupe alkyle, alcoxy ou halogéno,

ou bien

d)

R'$_1$ représente un atome d'hydrogène, un groupe alkyle ou benzyle, qui peut être substitué par un reste alkyle, alcoxy ou halogéno,

R'$_2$ représente un groupe phényle,

R'$_3$ représente un groupe alkyle ou phényle, et

R'$_4$ représente un atome d'hydrogène, un groupe alkyle, alcoxy ou halogéno, et

le terme "alkyle", lorsqu'il n'est pas cité spécialement, représente un groupe alkyle en $C_1$ à $C_{12}$, et le terme "alcoxy" représente un groupe alcoxy en $C_1$ à $C_{12}$, et les restes alkyles peuvent être substitués par du chlore, par un reste cyano ou carboxy.

**5.** Bis-indolyl-éthylènes selon la revendication 4, caractérisés en ce que :

R'$_1$ représente un atome d'hydrogène, un groupe alkyle ou benzyle,

R'$_2$ représente un groupe phényle substitué par un reste alkyle, alcoxy ou halogéno,

R'$_3$ représente un atome d'hydrogène ou un groupe alkyle ou phényle, et

R'$_4$ représente un atome d'hydrogène, un groupe alkyle, alcoxy ou halogéno, et

les restes alkyles et alcoxy ont 1 à 12 atomes de carbone.

**6.** Bis-indolyl-éthylènes selon la revendication 4, caractérisés en ce que :

R'$_1$ représente un atome d'hydrogène ou un groupe alkyle ou benzyle,

R'$_2$ représente un groupe phényle,

R'$_3$ représente un groupe alkyle ou phényle, et

R'$_4$ représente un atome d'hydrogène, un groupe alkyle, alcoxy ou halogéno, et

les restes alkyles et alcoxy comportent 1 à 12 atomes de carbone.